**Europäisches Patentamt**

**European Patent Office**

**Office européen des brevets**

(19)

(11) Veröffentlichungsnummer : **0 087 018**
**B1**

(12) **EUROPÄISCHE PATENTSCHRIFT**

(45) Veröffentlichungstag der Patentschrift :
30.12.86

(51) Int. Cl.⁴ : **A 61 B 17/04**

(21) Anmeldenummer : 83100950.1

(22) Anmeldetag : 02.02.83

(54) **Entlastungsnahtbesteck für die Chirurgie.**

(30) Priorität : 20.02.82 DE 3206279

(43) Veröffentlichungstag der Anmeldung :
31.08.83 Patentblatt 83/35

(45) Bekanntmachung des Hinweises auf die Patenterteilung : 30.12.86 Patentblatt 86/52

(84) Benannte Vertragsstaaten :
AT BE CH DE FR IT LI LU NL SE

(56) Entgegenhaltungen :
DE-A- 1 000 329
DE-A- 2 157 529
FR-A- 2 453 632
US-A- 2 199 025
US-A- 3 540 452
US-A- 3 931 821
CHIRURG, Band 50, 1979, Seite 188, Springer Verlag, W. MÜLLER: "Fixation von Nahtplatten mit drahtarmierten Redon-Drainagen zur Vermeidung des Platzbauches"

(73) Patentinhaber : INTERMEDICAT GMBH
Gerliswilstrasse 74
CH-6020 Emmenbrücke (CH)

(72) Erfinder : Sichler, Benno
Blumenstrasse 8
D-3508 Melsungen (DE)
Erfinder : Heinemann, Dieter
Birkenweg 6
D-3508 Melsungen (DE)
Erfinder : Meil, Gerhard
Guntershäuser Strasse 28
D-3501 Fuldabrück 1 (DE)
Erfinder : Fröhlich, Hans-Dieter
Müllersweg 8
D-3509 Spangenberg 3 (DE)

(74) Vertreter : von Kreisler, Alek, Dipl.-Chem. et al
Deichmannhaus am Hauptbahnhof
D-5000 Köln 1 (DE)

**Beschreibung**

Die Erfindung betrifft ein Entlastungsnahtbesteck für die Chirurgie, mit zwei mit Polsterplatten unterlegten relativ steifen länglichen Auflageplatten, welche von ihrer Längsmittellinie ausgehende, schräg zur Seitenkante der Auflageplatte bzw. Polsterplatte verlaufende, zu einer Seite hin frei auslaufende Schlitze aufweisen, und mit einem an mindestens einem Ende mit einer Nadel verbundenen Draht, welcher seitlich in die Schlitze einführbar ist.

Ein Entlastungsnahtbesteck dieser Art ist bekannt aus der Zeitschrift « CHIRURG », Band 50, 1979, Seite 188, Springer-Verlag. Dieses Entlastungsnahtbesteck weist zwei starre Auflageplatten auf, die mit gleich geformten separaten Polsterplatten unterlegt werden. Die Auflageplatten und die Polsterplatten haben jeweils von einer Seitenkante ausgehende, parallele schräge Schlitze, durch die der Draht seitlich eingeführt werden kann. Weitere Schlitze befinden sich an den Stirnseiten. Der Faden muß stets aus derselben Richtung schräg zur Auflageplatte in einen Schlitz eingeführt werden. Dies hat zur Folge, daß die Auflageplatten (und die darunter liegenden Polsterplatten) sich während des seitlichen Einführens des Fadens in Längsrichtung verschieben können. Außerdem können zu Beginn des Nahtvorganges Verschiebungen zwischen den Polsterplatten und den Auflageplatten stattfinden. Die Richtung, in der die Auflageplatten durch das seitliche Einführen des Drahtes verschoben werden, ist für alle Schlitze gleich.

Aus DE-A-2 157 529 ist ein chirurgischer Knopf bekannt, der aus einer runden Platte mit angeklebtem Polster besteht. Durch die Platte und das Polster geht ein Loch hindurch, durch welches ein Draht oder Faden gefädelt werden kann. Seitliche Schlitze sind hierbei nicht vorgesehen.

Der Erfindung liegt die Aufgabe zugrunde, ein Entlastungsnahtbesteck der eingangs genannten Art zu schaffen, dessen Handhabung für die Chirurgen einfacher ist und das vielfältigere Möglichkeiten der Drahteinführung in die Schlitze bietet.

Die Lösung dieser Aufgabe besteht erfindungsgemäß darin, daß jede Auflageplatte mit der zugehörigen Polsterplatte fest verbunden ist und daß die Schlitze paarweise derart angeordnet sind, daß zwei benachbarte Schlitze einen spitzen Winkel einschließen und nahe nebeneinander in die Seitenkante münden.

Bei dem erfindungsgemäßen Entlastungsnahtbesteck ist die Polsterplatte mit der zugehörigen Auflageplatte fest verbunden, so daß beide Platten sich nicht gegeneinander verschieben können. Der Chirurg muß also nicht mit vier Platten hantieren, sondern lediglich mit zwei Platten, was seine Arbeit sehr erleichtert. Die Schlitzanordnung in den Platten ermöglicht je nach Länge der Wunde eine Anpassung des Drahtabstandes und damit eine individuelle Entlastung des Gewebes. Dies hat darüber hinaus Vorteile für das Einführen der Drähte in die Schlitze, insbesondere dann, wenn der zwischen den benachbarten Schlitzen gebildete Keil gegenüber der Seitenkante zurückgesetzt ist. In diesem Fall ergibt sich an der Mündungsstelle der beiden Schlitze eine vergrößerte Einführöffnung.

Bei einer zweckmäßigen Ausführungsform der Erfindung ist die Auflageplatte in einer Vertiefung der Polsterplatte wenigstens teilweise versenkt untergebracht. Die Auflageplatte und/oder die Polsterplatte haben zweckmäßigerweise abgerundete Kanten, um die Gefahr von Entzündungen der Haut durch Kantenreibung zu vermeiden.

Gemäß einer bevorzugten Ausführungsform der Erfindung besteht die Seele des Drahtes aus zahlreichen Monofilen und sie ist mit einer Kunststoffummantelung umhüllt. Dadurch ist eine hohe Flexibilität des Drahtes bei gleichzeitiger hoher Zugfestigkeit, Gewebeverträglichkeit und nicht schneidendem Gewebedurchzug sichergestellt. Kapillarwirkung ist nicht gegeben.

Vorzugsweise ist die Seele des Drahtes in einer Klemmhülse der Nadel festgeklemmt, wobei der Außendurchmesser der Klemmhülse annähernd gleich groß ist wie der Außendurchmesser der Umhüllung. Auf diese Weise entsteht zwischen Nadel und Draht ein absatzfreier Übergang.

Die Umhüllung fluchtet außen mit der Klemmhülse der Nadel, so daß bei der Herstellung der Entlastungsnaht unnötige Beschädigungen des Gewebes vermieden werden.

Im folgenden werden Ausführungsbeispiele der Erfindung unter Bezugnahme auf die Zeichnungen näher erläutert.

Es zeigen :

Figur 1   eine Ansicht der Verbundplatte,

Figur 2   eine Darstellung des aus zwei Verbundplatten und einem Draht bestehenden Entlastungsnahtbestecks im angebrachten Zustand,

Figur 3   einen Längsschnitt durch die Verbundplatte nach Fig. 1,

Figur 4   einen Längsschnitt durch eine weitere Ausführungsform einer Verbundplatte,

Figur 5   einen Längsschnitt durch eine dritte Ausführungsform einer Verbundplatte,

Figuren 6, 7 und 8 Schnitte durch den Kantenbereich weiterer Ausführungsformen von Verbundplatten,

Figur 9   eine Darstellung des Drahtes mit an beiden Enden angebrachten Nadeln, und

Figur 10   einen Längsschnitt durch den Übergangsbereich zwischen Draht und Nadel.

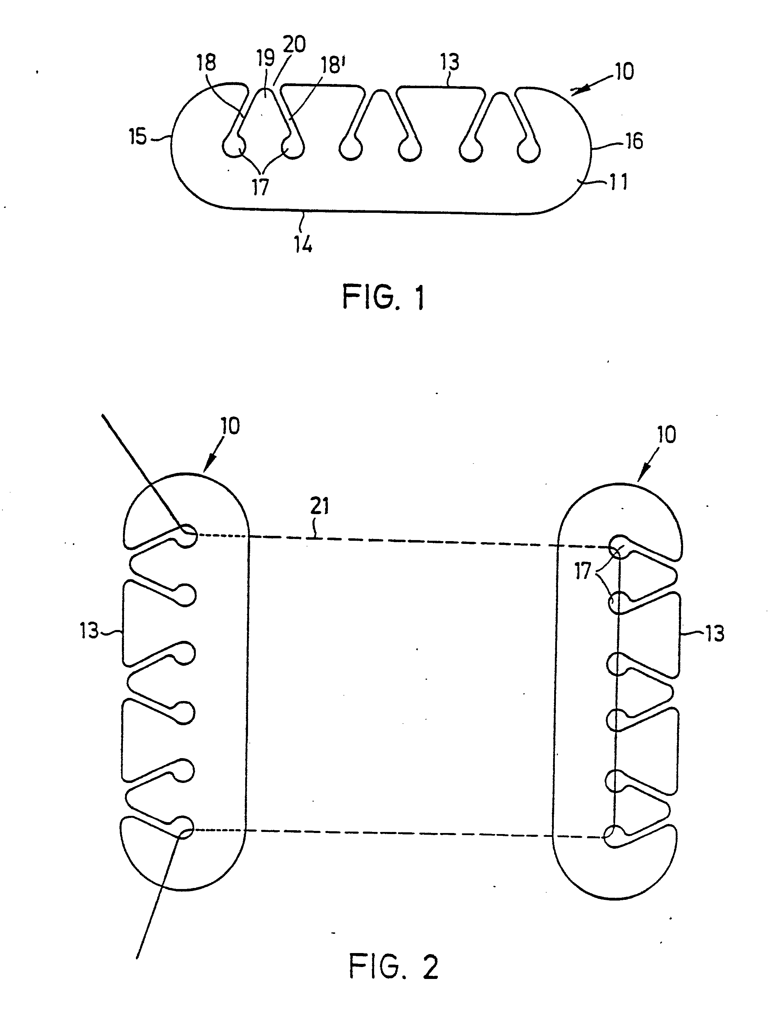

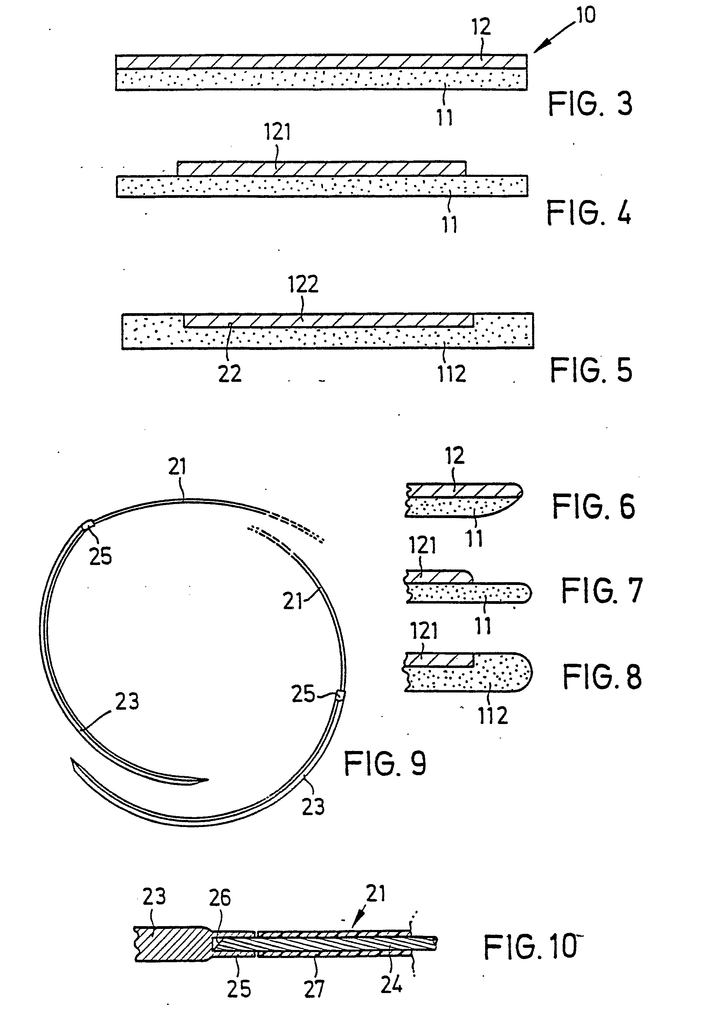

Die in den Fig. 1 und 3 dargestellte Verbundplatte 10 besteht aus einer Polsterplatte 11 und einer Auflageplatte 12. Die die Verbundplatte 10 bildenden Platten 11 und 12 sind einander deckungsgleich. Die Verbundplatte 10 ist langgestreckt und weist parallele Seitenkanten 13, 14 und halbkreisförmige Stirnseiten 15, 16 auf. Auf der Längsmittellinie der Verbundplatte 10 sind äquidistant mehrere Löcher 17 angeordnet, die über die gesamte Stärke der Verbundplatte durch diese hindurchgehen. Von jedem der kreisrunden Löcher 17 erstreckt sich ein Schlitz 18 bzw. 18', der sich ebenfalls über die gesamte Stärke der Verbundplatte erstreckt, bis zu der einen Seitenkante 13, wo er frei nach außen ausläuft. Die Schlitze 18 und 18' sind einander paarweise derart zugeordnet, daß sie einen Keil 19 mit spitzem Winkel einschließen. Das Ende dieses Keiles ist gegenüber der Seitenkante 13 geringfügig zurückgesetzt, so daß die äußeren Enden der ein Schlitzpaar bildenden Schlitze 18 und 18' einen relativ großflächigen Mündungsbereich 20 bilden, der das seitliche Einschieben eines Drahtes in einen der Schlitze erleichtert.

Bei dem Ausführungsbeispiel der Fig. 1 sind insgesamt 6 Löcher 17 vorhanden, so daß sich insgesamt drei Schlitzpaare 18, 18' ergeben.

In Fig. 2 sind die beiden zu dem Entlastungsnahtbesteck gehörenden Verbundplatten 10 dargestellt. Diese Verbundplatten 10 werden mit gegenseitigem Abstand zu beiden Seiten der (nicht dargestellten) Operationsnaht angeordnet und mit der Auflageplatte 11 nach unten auf die Haut des Patienten aufgelegt. Hierbei weisen die Seitenkanten 13, in die die Schlitze 18 bzw. 18' einmünden, jeweils nach außen, d. h. voneinander fort.

In einige der Schlitze 18 bzw. 18' wird ein Draht 21, der nachfolgend noch erläutert wird, seitlich eingeschoben. Dieser Draht 21 wird mit einer Nadel durch die Bauchdecke des Patienten hindurchgestochen und z. B. wie in der in Fig. 2 dargestellt verlegt.

Bei dem Ausführungsbeispiel nach Fig. 4 nimmt die Auflageplatte 121 eine kleinere Fläche ein als die Polsterplatte 11. Die Löcher 17 und die Schlitze 18, 18' erstrecken sich jedoch, ebenso wie bei dem Ausführungsbeispiel der Fig. 1 bis 3, durch beide Platten 11 und 121 hindurch.

Bei dem Ausführungsbeispiel der Fig. 5 weist die Polsterplatte 112 an ihrer Oberseite eine Ausnehmung oder Vertiefung 22 auf, in die die Auflageplatte 122 passend eingelegt ist. Die Fläche der Polsterplatte 112 ist hierbei selbstverständlich größer als diejenige der Auflageplatte 122 und auch die Stärke der Polsterplatte ist größer.

Nach Fixierung der unter Spannung stehenden Unterstützungsnaht können durch die Auflage der Platten auf der Haut Unannehmlichkeiten für den Patienten durch die Kantenreibung bzw. durch Nekrosenbildung der Platten entstehen. Daher sollte mindestens die Kante an dem unteren Rand der Polsterplatte abgerundet sein. In den Fig. 6, 7 und 8 sind derartig abgerundete Kanten für die Ausführungsbeispiele der Fig. 3 bis 5 dargestellt. Zusätzlich sind gemäß Fig. 6 und 7 auch noch die oberen Seitenkanten der Auflageplatten 12 bzw. 121 abgerundet.

Bei den dargestellten Ausführungsbeispielen bestehen sowohl die Polsterplatten als auch die Auflageplatten aus dem Grundmaterial Polyäthylen. Die Auflageplatten 12, 121, 122 bestehen jedoch aus einem Vollmaterial, während die Polsterplatten 11, 112 aus Schaumstoff bestehen.

Jede Polsterplatte ist mit der zugehörigen Auflageplatte vollflächig verbunden, z. B. mittels Heißlaminat durch Flammkaschieren. Sowohl die starren Platten als auch die Polsterplatten können mit Löchern 17 und Schlitzen 18, 18' einzeln vorgefertigt sein (Spritzguß, Stanzarbeit o. ä.) und die Verbindung erfolgt anschließend aus den einzelnen Platten, z. B. mittels Heißlaminat durch Flammkaschieren oder andere Klebetechniken.

Je nach Fertigungsprozeß können auch die Konturen, die Löcher 17 und die Schlitze 18 und 18' erst nach Verbindung großflächiger Plattenformate gestanzt werden, wobei anschließend Glätten der Stanzfläche erfolgt (z. B. thermisch). Die Ränder der Löcher und Schlitze sind nach außen hin abgeschrägt.

In den Fig. 9 und 10 ist der Draht 21 dargestellt. Dieser Draht, der flexibel ist, weist an seinen beiden Enden je einer gebogene starre Nadel 23 auf. Die Nadeln 23 sind durch die Drahtseele 24 miteinander verbunden. Jede Nadel ist an ihrem rückwärtigen Ende mit einer zylindrischen Klemmhülse 25 versehen, deren Querschnittsfläche etwa derjenigen des übrigen Bereichs der Nadel entspricht. In die Klemmhülse 25 wird das Ende der Drahtseele 24 eingeschoben und verklemmt. Die Schneidkante 26 der Drahtseele verläuft nicht nur rechtwinklig zur Achse der Drahtseele, sondern kann auch unter einem Winkel von 80 bis 60° verlaufen, um eine bessere Armierung zu erzielen.

Die Drahtseele 24 besteht z. B. aus 19 Monofilamenten, von denen sieben einen Drahtkern und zwölf einen Drahtmantel bilden. Die Drahtseele ist mit einer aus Kunststoff bestehenden Umhüllung 27 mit einer Stärke von 0,35 mm umgeben. Die Umhüllung endet jedoch anstoßend an die Klemmhülsen 25, so daß in den Klemmhülsen 25 nur die Drahtseele 24 eingeklemmt wird. Die Stärke der Klemmhülse 25 entspricht etwa derjenigen der Umhüllung 27, so daß an der Übergangsstelle zwischen der Umhüllung 27 und der Klemmhülse 25 keine Stoßstelle gebildet wird.

## Patentansprüche

1. Entlastungsnahtbesteck für die Chirurgie, mit zwei mit Polsterplatten (11) unterlegten relativ steifen länglichen Auflageplatten (12), welche von ihrer Längsmittellinie ausgehende, schräg zur Seitenkante (13) der Auflageplatte (12) bzw. Polsterplatte (11) verlaufende, zu einer Seite hin frei auslaufende Schlitze (18) aufweisen, und mit einem an mindestens einem Ende mit einer Nadel verbundenen Draht, welcher seitlich in die Schlitze einführbar ist, dadurch gekennzeichnet, daß jede Auflageplatte (12) mit der zugehörigen Polsterplatte (11) fest verbunden ist und daß die Schlitze (18, 18') paarweise derart angeordnet sind, daß zwei benachbarte Schlitze (18, 18') einen spitzen Winkel einschließen und nahe nebeneinander in die Seitenkante (13) münden.

2. Entlastungsnahtbesteck nach Anspruch 1, dadurch gekennzeichnet, daß der zwischen den benachbarten Schlitzen (18, 18') gebildete Keil (19) gegenüber der Seitenkante (13) zurückgesetzt ist.

3. Entlastungsnahtbesteck nach Anspruch 1 oder 2, dadurch gekennzeichnet, daß die Auflageplatte (122) in einer Vertiefung (22) der Polsterplatte (112) wenigstens teilweise versenkt untergebracht ist.

4. Entlastungsnahtbesteck nach einem der Ansprüche 1 bis 3, dadurch gekennzeichnet, daß die Auflageplatte (12) und/oder die Polsterplatte (11) abgerundete Kanten aufweisen.

5. Entlastungsnahtbesteck nach einem der Ansprüche 1 bis 4, dadurch gekennzeichnet, daß die Seele (24) des Drahtes (21) aus zahlreichen Monofilen besteht und mit einer Kunststoffummantelung (27) umhüllt ist.

6. Entlastungsnahtbesteck nach Anspruch 5, dadurch gekennzeichnet, daß die Seele (24) des Drahtes (21) in einer Klemmhülse (25) der Nadel (23) festgeklemmt ist und daß der Außendurchmesser der Klemmhülse (25) annähernd gleich groß ist wie der Außendurchmesser der Umhüllung (27).

## Claims

1. Surgical suture relief set comprising two relatively rigid elongated support plates (12) which are lined with padding plates (11) and which, starting from their longitudinal center line include slots extending obliquely to the lateral edge (13) of the support plate (12) or padding plate (11) to freely end towards one side, and a wire whose one end at least is connected to a needle and which may be introduced laterally into the slots, characterized in that each support plate (12) is integrally joined to the associated padding plate (11) and that the slots (18, 18') are arranged in pairs so that two adjacent slots (18, 18') include an acute angle and end closely side by side in the lateral edge.

2. Surgical suture relief set as defined in claim 1, characterized in that the key (19) formed intermediate adjacent slots (18, 18') is recessive relative to the lateral edge (13).

3. Surgical suture relief set as defined in claim 1 or 2, characterized in that the support plate (122) is provided to be at least partly embedded in a recess (22) of the padding plate (112).

4. Surgical suture relief set as defined in one of claims 1 to 3, characterized in that the support plate (12) and/or the padding plate (11) are provided with edges of the rounded-off type.

5. Surgical suture relief set as defined in one of claims 1 to 4, characterized in that the core (24) of the wire (21) is formed of a plurality of monofils and wrapped by a plastic sheathing (27).

6. Surgical suture relief set as defined in claim 5, characterized in that the core (24) of the wire (21) is tightened in a clamping sleeve (25) of the needle (23) and that the outer diameter of the clamping sleeve (25) is approximately equal to the outer diameter of the sheathing (27).

## Revendications

1. Ensemble de détente de suture chirurgicale, comportant deux plaques de support allongées, relativement rigides (12), qui sont soutenues par deux plaques de rembourrage (11) et comportent des fentes (18) qui s'étendent à partir de l'axe médian longitudinal des plaques et sont disposées obliquement par rapport au bord latéral (13) de la plaque de support (12) ou de la plaque de rembourrage (11) et débouchent librement d'un côté, et comportant un fil relié au moins au niveau d'une extrémité à une aiguille et qui peut être introduit latéralement dans les fentes, caractérisé en ce que chaque plaque de support (12) est reliée rigidement à la plaque de rembourrage associée (11) et que les fentes (18, 18') sont disposées par couples de telle sorte que deux fentes voisines (18, 18') font entre elles un angle aigu et débouchent à proximité l'une de l'autre dans le bord latéral (13).

2. Ensemble de détente pour suture selon la revendication 1, caractérisé en ce que le coin (19), qui est formé entre les fentes (18, 18') voisines, est en retrait par rapport au bord latéral (13).

3. Ensemble de détente pour suture selon la revendication 1 ou 2, caractérisé en ce que la plaque de support (122) est logée, en étant au moins partiellement noyée, dans un renfoncement (22) de la plaque de rembourrage (112).

4. Ensemble de détente pour suture selon l'une des revendications 1 à 3, caractérisé en ce que la plaque de support (12) et/ou la plaque de rembourrage (12) comportent des bords arrondis.

5. Ensemble de détente pour suture selon l'une des revendications 1 à 4, caractérisé en ce que l'âme (24) du fil (21) est constituée par un grand nombre de monofils et est enveloppée par une gaine en matière plastique (27).

6. Ensemble de détente pour suture selon la revendication 5, caractérisé en ce que l'âme (24) du fil (21) est bloquée dans une douille de serrage (25) de l'aiguille (23) et que le diamètre extérieur de la douille de serrage (25) est approximativement égal au diamètre extérieur de la gaine (27).

FIG. 1

FIG. 2

1

FIG. 3

FIG. 4

FIG. 5

FIG. 6

FIG. 7

FIG. 8

FIG. 9

FIG. 10